# EUROPEAN PATENT SPECIFICATION

(11) **EP 2 870 961 B1**
(45) Date of publication and mention of the grant of the patent: **18.07.2018**
(21) Application number: 14191800.3
(22) Date of filing: 05.11.2014
(51) Int. Cl.: A61K 9/46, A61K 31/428

(54) **Effervescent formulations of pramipexole**
Brauseformulierungen von Pramipexol
Formulations effervescentes de pramipexole

(30) Priority: 06.11.2013 TR 201312846; 26.12.2013 TR 201315298
(43) Date of publication of application: 13.05.2015
(73) Proprietor: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR); Güner, Dicle, 34460 Istanbul (TR)
(74) Representative: Sevinç, Erkan

(56) References cited:
- EP-A1- 2 308 464
- WO-A1-2013/100871
- WO-A2-99/04765
- KR-A- 20120 134 545
- US-A1- 2010 204 259

## Description

### Field of Invention

The present invention relates to an effervescent formulation comprising pramipexole or a pharmaceutically acceptable salt, solvate or hydrate thereof and one or more pharmaceutically acceptable excipients.

### Background of Invention

Pramipexole is a nonergot dopamine agonist. The chemical name of pramipexole is (S)-2-amino-4,5,6,7-tetrahydro-6-(propylamino)benzothiazole and its chemical structure is shown in the Formula 1.

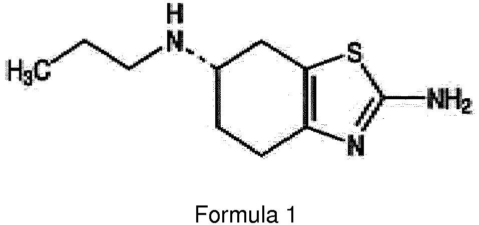

Pramipexole immediate release tablets are marketed under the name of Mirapex® and are indicated to be taken 3 times a day for oral administration and contain 0.125 mg, 0.25 mg, 0.5 mg, 1 mg, or 1.5 mg of pramipexole dihydrochloride monohydrate as active ingredient.

In prior art, pramipexole and its processes are described first in EP0186087B1 and it is known primarily for the treatment of schizophrenia and Parkinson's disease. There are also several patents and applications which disclose tablet formulations of pramipexole but none of them comprises effervescent formulations of pramipexole.

Effervescent tablets are designed to break down quickly and release carbon dioxide when dropped in liquid. They have gained considerable attention as a preferred alternative to conventional tablets and capsules due to their better patient compliance. Today, there are a growing number of people who cannot swallow tablets or capsules. On the other side, in effervescent formulations the active ingredient is already solubilized in water before its administration and liquid effervescent form is easier to take as compared to tablets or capsules.

In addition to ease of administration, many studies have demonstrated that effervescent formulations enhance absorption of a number of active ingredients and reduce the side effects of drug. They generally contain acidifying agents and alkalizing agents which react rapidly in the presence of water by releasing carbon dioxide such as carbonates or bicarbonates. Carbon dioxide emitted by the effervescent reaction can induce enhanced active-ingredient permeability due to an alteration of the paracellular pathway. Moreover, the low pH in the stomach can cause active ingredients to become denatured, lose activity, or cause them to remain inactive. Acidifying agent - alkalizing agent couples, however, can buffer the water-active solution so that the stomach pH increases and thus prevent the degradation or inactivation of the active ingredient.

On the other side, these formulations should not be too rigid to dissolve in water but should have porous structure. However, this complex structure later causes stability problems by affecting from humidity. Moreover, unpleasant and bitter taste upon disintegration in water is problem that should be overcome. Therefore, it is needed to develop an effervescent formulation which is non-hygroscopic and which has a desired disintegration time and acceptable taste for patients.

In this invention, effervescent formulations comprising pramipexole or a pharmaceutically acceptable salt, solvate or hydrate thereof have been developed. To provide effervescent formulations with high stability, desired disintegration time and acceptable taste, one or more pharmaceutically acceptable excipient has been used.

### Description of the invention

The present invention provides an effervescent formulation comprising pramipexole or a pharmaceutically acceptable salt, solvate or hydrate thereof and one or more pharmaceutically acceptable excipient which is selected from the group consisting of acidifying agent, alkalizing agent, fillers, disintegrants, binders, lubricants, antioxidants, aromatic agents and sweeteners; and the ratio of the acidifying agents to the alkalizing agents is between 30:1 (w/w) and 1:30 (w/w), preferably between 15:1 (w/w) and 1:15 (w/w) and more preferably it is between 3:1 (w/w) and 1:3 (w/w). In one embodiment, the effervescent formulation is in the form of a tablet or a sachet.

In a preferred embodiment, pramipexole or a pharmaceutically acceptable salt, solvate or hydrate present in the effervescent formulation as pramipexole dihydrochloride monohydrate.

According to one embodiment, the amount of pramipexole dihydrochloride monohydrate is between 0.01 - 20.0%, preferably 0.01 - 10% and more preferably it is 0.01 - 5% by weight of total formulation.

Choice of acidifying agent - alkalizing agent couple and their ratio is crucial in terms of stability and disintegration time. The ratio of acidifying agent to alkalizing agent should be optimum to obtain a neutral solution without any residue and precipitation after tablet disintegration. High amount of acidifying agent causes low pH which may damage the gastrointestinal system. On the other side, high amount of alkalizing agent (especially hydrophobic agents) also causes unclear and precipitated solution and induces damage in gastrointestinal system. This ratio also affects disintegration time of effervescent formulation. In this invention, within a specific ratio of these two agents, desired neutral solution without any precipitation and with a desired disintegration time has been achieved up to 5 min. Moreover, by adjusting the ratio of acidifying agent to alkalizing agent, desired taste of effervescent solution has also been obtained in order to contribute aromatic agenttic agents used in the formulation.

According to this embodiment, the acidifying agent is selected from the group comprising fumaric acid, tartaric acid, citric acid, citric acid monohydrate, citric acid anhydrate, adipic acid, ascorbic acid, acetic acid, hydrochloric acid, malic acid, nitric acid, phosphoric acid, nicotinic acid, acetyl salicylic acid, sulfuric acid, acid salts (such as amino acid hydrochlorides, sodium citrates, sodium citrate dihydrate, disodium citrate, disodium dihydrogen citrate, sodium acid phosphate) and mixtures thereof, preferably it is fumaric acid.

Effervescent formulations should be porous and not too rigid to dissolve in water. However, this hygroscopic porous structure may cause stability problems by affecting from humidity, especially during the production, packaging or transportation. Therefore, acidifying agent - alkalizing agent couple should be non-hygroscopic to overcome stability problems.

According to this embodiment, fumaric acid has been used as an acidifying agent for this present invention. Fumaric acid which is a non-hygroscopic organic acid has stability to degradation by microorganisms as well as chemical stability. It also shows lubricating property while prevents stickness and improves compressibility.

In this embodiment, the amount of fumaric acid is in the range of 2 - 90 % and preferably 2 - 60 % by weight of total formulation.

According to one embodiment, the alkalizing agent is selected from the group comprising sodium glycine carbonate, ammonia solution, ammonium carbonate, diethanolamine, diisopropanolamine, potassium hydroxide, potassium bicarbonate, potassium carbonate, calcium carbonate, sodium bicarbonate, sodium borate, sodium carbonate, sodium hydroxide, trolamine and mixtures thereof, preferably it is sodium glycine carbonate.

In one embodiment, sodium glycine carbonate which reacts rapidly in the water by releasing carbon dioxide has been used as an alkalizing agent which improves gastric absorption of the drug. Sodium glycine carbonate is a complex of glycine and sodium carbonate. It is more soluble and less alkalinity in water than other alkalizing agents. It is water-soluble, heat resistant but moisture sensitive when used alone.

In this embodiment, the amount of sodium glycine carbonate is in the range of 2 - 90 % and preferably 2 - 60 % by weight of total formulation.

In the present invention, when fumaric acid and sodium glycine carbonate have been used together, a non-hygroscopic acidifying agent - alkalizing agent couple has been obtained which shows a synergistic effect on disintegration time, stability and compressibility of the formulation. It is suprisingly found that stability has also been increased by using fumaric acid and sodium glycine carbonate together, although sodium glycine carbonate is moisture sensitive. In addition, by using fumaric acid and sodium glycine carbonate, desired disintegration time has been achieved up to 5 min. Furthermore, the formulation of the present invention has shown high compressibility properties due to high compressibility characteristics of sodium glycine carbonate and lubricating property of fumaric acid.

According to this embodiment, the ratio of fumaric acid to sodium glycine carbonate is between 30:1 (w/w) and 1:30 (w/w), preferably between 15:1 (w/w) and 1:15 (w/w) and more preferably it is between 3:1 (w/w) and 1:3 (w/w).
The stability assay has been performed by HPLC with gradient mobile phase at a wavelength of UV 264 nm at 40 ± 10 ºC of column temperature.

Suitable fillers may include but not limited to xylitol, trehalose, sorbitol, lactose, sugars, mannitol, sucrose, inorganic salts, calcium salts, polysaccharides, dextrose, dicalsium phosphate, sodium chloride, dextrates, lactitol, maltodextrin, sucrose-maltodextrin mixtures, heavy magnesium carbonate, izomalt and mixtures thereof.

Suitable disintegrants may include but not limited to crospovidone, polyvinylpyrrolidone, alginic acid and alginates, microcrystalline cellulose, sodium starch glycolate, magnesium aluminum silicate, sodium lauryl sulfate, sodium carboxymethyl cellulose, croscarmellose sodium, cross-linked polyvinylpyrrolidone (PVP), vinylpyrrolidone-vinyl acetate copolymers (Kollidon VA64), carboxymethyl cellulose calcium, guar gum, low-substituted hydroxypropyl cellulose, polyacrylin potassium, poloxamer, povidone, sodium alginate, sodium glycine carbonate, sodium lauryl sulfate, ion-exchange resins or the mixtures thereof.

Suitable binders may include but not limited to sugars, glucose syrup, natural gums, starch, gelatin, polyvinylpyrrolidone, polymethacrylates; collagen, proteins such as gelatin; agar, alginate, sodium alginate, pectin, starch, carboxymethyl cellulose, hydroxypropyl cellulose, methyl cellulose and similar semi-synthetic polymers; carbomer, poloxamer, polyacrylamide, polyvinyl alcohol and similar synthetic polymers; aluminum hydroxide, bentonite, laponite and other inorganic substances; starch mucilage, acacia mucilage, polydextrose, polyethylene oxide, or the mixtures thereof. Suitable lubricants may include but not limited to polyethylene glycol (PEG), sodium stearyl fumarate, magnesium stearate, sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof.

Suitable antioxidants may include but not limited to butyl hydroxyl anisole, butyl hydroxyl toluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium dithionite, gentisic acid ethanolamine, monosodium glutamate, sodium formaldehyde sulfoxylate, alpha tocopherol, or the mixtures thereof.

Suitable aromatic agenttic agents may include but not limited to fruit aromatic agents such as orange, banana, strawberry, cherry, wild cherry, lemon, etc., and other aromatic agents such as cardamom, anis, mint, menthol, vanillin, and the mixtures thereof.

Suitable sweeteners may include but not limited to sucralose, sodium cyclamate, thaumatin, mogroside, inuline, erythritol, glycyrrhizin, monosodium glycyrrhizinate, monoamonium glycyrrhizinate, isomalt, glycerine, dextrose or mixtures thereof.

### Example 1: effervescent tablet (wet granulation)

| **Ingredients** | **Amount (%)** |
|---|---|
| pramipexole dihydrochloride monohydrate | 0.01 - 10.0 |
| microcrystalline cellulose | 2.00 - 50.00 |
| polyvinylpyrrolidone | 0.10 - 25.00 |
| butyl hydroxy anisole | 0.01 - 2.00 |
| butyl hydroxy toluene | 0.01 - 2.00 |
| sodium glycine carbonate | 2.00 - 60.00 |
| fumaric acid | 2.00 - 60.00 |
| thaumatin | 0.10 - 2.00 |
| polyethylene glycol (PEG 6000) | 0.50 - 10.00 |
| water/alcohol | 0.50- 90.00 |
| aromatic agent | 0.10 - 5.00 |

The production of the formulation is carried out as follows: Microcrystalline cellulose, fumaric acid, sodium glycine carbonate are sieved and mixed. Butyl hydroxy anisole, butyl hydroxy toluene, pramipexole dihydrochloride monohydrate and polyvinylpyrrolidone are dissolved in alcohol and wet granulation is performed with this solution. Wet granules are sieved and dried. Dried granules are sieved. Thaumatin and aromatic agent are added to granules and mixed until homogenous mixture is observed. Polyethylene glycol was added to total mixture and the mixture is pressed into tablets.

### Example 2: effervescent tablet (direct compression)

| **Ingredients** | **Amount (%)** |
|---|---|
| pramipexole dihydrochloride monohydrate | 0.01 - 10.0 |
| microcrystalline cellulose | 2.00 - 50.00 |
| crospovidone | 1.00 - 30.00 |
| erythritol | 5.00- 50.00 |
| vinylpyrrolidone-vinyl acetate copolymers (Kollidon VA 64) | 1.00- 30.00 |
| sodium glycine carbonate | 2.00 - 60.00 |
| fumaric acid | 2.00 - 60.00 |
| sucralose | 0.10 - 2.00 |
| polyethylene glycol (PEG 6000) | 0.25 - 2.00 |
| aromatic agent | 0.10- 5.00 |

The production of the formulation is carried out as follows: pramipexole dihydrochloride monohydrate, microcrystalline cellulose, crospovidone, erythritol, Kollidon VA 64, fumaric acid, sodium glycine carbonate, sucralose and aromatic agent are mixed until homogenous mixture is observed. The mixture and polyethylene glycol are mixed and pressed into tablets.

### Example 3: effervescent sachet (drying and direct filling)

| **Ingredients** | **Amount (%)** |
|---|---|
| pramipexole dihydrochloride monohydrate | 0.01 - 10.0 |
| xylitol | 5.00 - 50.00 |
| sodium glycine carbonate | 2.00 - 60.00 |
| fumaric acid | 2.00 - 60.00 |
| sodium citrate dihydrate | 2.00 - 60.00 |
| tartaric acid | 2.00 - 60.00 |
| sucralose | 0.10 - 2.0 |
| thaumatin | 0.10 - 2.0 |
| aromatic agent | 0.10 - 5.00 |

The production of the formulation is carried out as follows: pramipexole dihydrochloride monohydrate, fumaric acid, tartaric acid, sodium glycine carbonate and sodium citrate dihydrate sieved and mixed. The mixture is dried in oven at 105 ºC. Dried granules are sieved. Xylitol, sucralose, thaumatin and aromatic agent are added to dried granules and mixed till a homogenous mixture is observed. The powder mixture is filled into sachet bags.

### Example 4: effervescent sachet (wet granulation)

| **Ingredients** | **Amount (%)** |
|---|---|
| pramipexole dihydrochloride monohydrate | 0.01 - 10.0 |
| trehalose | 5.00 - 50.00 |
| polyvinylpyrollidone | 0.10 - 25.00 |
| sodium glycine carbonate | 2.00 - 60.00 |
| fumaric acid | 2.00 - 60.00 |
| sodium citrate | 2.00 - 60.00 |
| sucralose | 0.10 - 2.00 |
| mogroside | 0.10 - 2.00 |
| water/alcohol | 0.50- 90.00 |
| aromatic agent | 0.10 - 5.00 |

The production of the formulation is carried out as follows: the solution of polyvinylpyrrolidone in alcohol/alcohol-water mixture is prepared. Pramipexole dihydrochloride monohydrate and trehalose are mixed and granulated with polyvinylpyrrolidone solution respectively. Granules are dries in oven. Dried granules are sieved. Fumaric acid, sodium glycine carbonate, sodium citrate, sucralose, magroside and aromatic agent are added to granules and mixed till a homogenous mixture is observed. The powder mixture is filled into sachet bags.

### Example 5: effervescent sachet (direct filling)

| **Ingredients** | **Amount (%)** |
|---|---|
| pramipexole dihydrochloride monohydrate | 0.01 - 10.0 |
| trehalose | 5.00 - 50.00 |
| citric acid anhydrate | 2.00 - 60.00 |
| sorbitol | 2.00 - 60.00 |
| sodium glycine carbonate | 2.00 - 60.00 |
| fumaric acid | 2.00 - 60.00 |
| sucralose | 0.10 - 2.00 |
| sodium cyclamate | 0.10 - 2.00 |
| aromatic agent | 0.10 - 5.00 |

The production of the formulation is carried out as follows: Pramipexole dihydrochloride monohydrate, citric acid anhydrate, sorbitol, fumaric acid, sodium glycine carbonate, sucralose, sodium cyclamate, trehalose and aromatic agent are sieved and mixed till a homogenous mixture is observed. The powder mixture is filled into sachet bags.

All process are performed under the conditions as follows: at 25 ºC with % 40 RH(relative humudity), preferably at 20 ºC with % 25 RH(relative humidity) and more preferably at 15-20 ºC with % 10-20 RH(relative humidity).

## Claims

1. An effervescent formulation comprising pramipexole or a pharmaceutically acceptable salt, solvate or hydrate thereof and one or more pharmaceutically acceptable excipient which is selected from the group consisting of acidifying agent, alkalizing agent, fillers, disintegrants, binders, lubricants, antioxidants, aromatic agents and sweeteners; and the ratio of the acidifying agents to the alkalizing agents is between 30:1 (w/w) and 1:30 (w/w), preferably between 15:1 (w/w) and 1:15 (w/w) and more preferably it is between 3:1 (w/w) and 1:3 (w/w).

2. The effervescent formulation according to claim 1, wherein the acidifying agent is selected from the group comprising fumaric acid, tartaric acid, citric acid, citric acid monohydrate, citric acid anhydrate, adipic acid, ascorbic acid, acetic acid, hydrochloric acid, malic acid, nitric acid, phosphoric acid, nicotinic acid, acetyl salicylic acid, sulfuric acid, acid salts amino acid hydrochlorides, sodium citrates, sodium citrate dihydrate, disodium citrate, disodium dihydrogen citrate, sodium acid phosphate and mixtures thereof, preferably it is fumaric acid.

3. The effervescent formulation according to claim 2, wherein the amount of fumaric acid is in the range of 2 - 90 % and preferably 2 - 60 % by weight of total formulation.

4. The effervescent formulation according to claim 1, wherein the alkalizing agent is selected from the group comprising sodium glycine carbonate, ammonia solution, ammonium carbonate, diethanolamine, diisopropanolamine, potassium hydroxide, potassium bicarbonate, potassium carbonate, calcium carbonate, sodium bicarbonate, sodium borate, sodium carbonate, sodium hydroxide, trolamine and mixtures thereof, preferably it is sodium glycine carbonate.

5. The effervescent formulation according to claim 4, wherein the amount of sodium glycine carbonate is in the range of 2 - 90 % and preferably 2 - 60 % by weight of total formulation.

6. The effervescent formulation according to any preceding claim, in the form of a tablet or a sachet.

7. The effervescent formulation according to any preceding claim comprising;
a) 0.01 - 10.0% by weight of pramipexole dihydrochloride monohydrate
b) 2.00 - 50.00 % by weight of microcrystalline cellulose
c) 1.00 - 25.00% by weight of polyvinylpyrrolidone
d) 0.01 - 2.00 % by weight of butyl hydroxy anisole
e) 0.01 - 2.00 % by weight of butyl hydroxy toluene
f) 2.00 - 60.00 % by weight of sodium glycine carbonate
g) 2.00 - 60.00 % by weight of fumaric acid
h) 0.10 - 2.00 % by weight of thaumatin
i) 0.50 - 10.00 % by weight of polyethylene glycol
j) 2.00 - 50.00 % by weight of sodium bicarbonate
k) 0.50- 90.00 % by weight of water/alcohol
l) 0.10 - 5.00 % by weight of aromatic agent

8. The effervescent formulation according to any preceding claim comprising;
a) 0.01 - 10.0% by weight of pramipexole dihydrochloride monohydrate
b) 2.00 - 50.00 % by weight of microcrystalline cellulose
c) 1.00 - 30.00 % by weight of crospovidone
d) 5.00- 50.00 % by weight of erythritol
e) 1.00- 30.00 % by weight of vinylpyrrolidone-vinyl acetate copolymers
f) 2.00 - 60.00 % by weight of sodium glycine carbonate
g) 2.00 - 60.00 % by weight of fumaric acid
h) 0.10 - 2.00 % by weight of sucralose
i) 0.25 - 2.00 % by weight of polyethylene glycol
j) 0.10 - 5.00 % by weight of aromatic agent

9. The effervescent formulation according to any preceding claim comprising;
a) 0.01 - 10.0% by weight of pramipexole dihydrochloride monohydrate
b) 5.00- 50.00 % by weight of xylitol
c) 2.00 - 60.00 % by weight of sodium glycine carbonate
d) 2.00 - 60.00 % by weight of fumaric acid
e) 2.00 - 60.00 % by weight of sodium citrate dehydrate
f) 2.00 - 60.00 % by weight of tartaric acid
g) 0.10 - 2.00 % by weight of sucralose
h) 0.10 - 2.00 % by weight of thaumatin
i) 0.10 - 5.00 % by weight of aromatic agent

10. The effervescent formulation according to any preceding claim comprising;
a) 0.01 - 10.0% by weight of pramipexole dihydrochloride monohydrate
b) 5.00- 50.00 % by weight of trehalose
c) 0.10 - 25.00 % by weight of polyvinylpyrollidone
d) 2.00 - 60.00 % by weight of sodium glycine carbonate
e) 2.00 - 60.00 % by weight of fumaric acid
f) 2.00 - 60.00 % by weight of sodium citrate
g) 0.10 - 2.00 % by weight of sucralose
h) 0.10 - 2.00 % by weight of mogroside
i) 0.50- 90.00 % by weight of water/alcohol
j) 0.10 - 5.00 % by weight of aromatic agent

11. The effervescent formulation according to any preceding claim comprising;
a) 0.01 - 10.0% by weight of pramipexole dihydrochloride monohydrate
b) 5.00- 50.00 % by weight of trehalose
c) 0.10 - 25.00 % by weight of citric acid anhydrate
d) 2.00 - 60.00 % by weight of sodium glycine carbonate
e) 2.00 - 60.00 % by weight of fumaric acid
f) 2.00 - 60.00 % by weight of sorbitol
g) 0.10 - 2.00 % by weight of sucralose
h) 0.10 - 2.00 % by weight of sodium cyclamate
i) 0.10 - 5.00 % by weight of aromatic agent

## Patentansprüche

1. Brauseformulierung, welche Pramipexol oder ein pharmazeutisch verträgliches Salz, Solvat oder Hydrat davon enthält und einen oder mehrere pharmazeutisch verträgliche(n) Hilfsstoff(e), welche(r) ausgewählt ist/sind aus der Gruppe bestehend aus Säuerungsmitteln, Alkalisierungsmitteln, Füllstoffen, Sprengmitteln, Bindern, Schmiermitteln, Antioxidantien, aromatischen Mitteln und Süßstoffen, wobei das Verhältnis der Säuerungsmittel zu den Alkalisierungsmitteln zwischen 30:1 (w/w) und 1:30 (w/w) liegt, vorzugsweise zwischen 15:1 (w/w) und 1:15 (w/w) und noch bevorzugter zwischen 3:1 (w/w) und 1:3 (w/w).

2. Brauseformulierung nach Anspruch 1, wobei das Säuerungsmittel ausgewählt ist aus der Gruppe bestehend aus Fumarsäure, Weinsäure, Zitronensäure, Zitronensäuremonohydrat, Zitronensäureanhydrat, Adipinsäure, Ascorbinsäure, Essigsäure, Salzsäure, Apfelsäure, Salpetersäure, Phosphorsäure, Nikotinsäure, Acetylsalicylsäure, Schwefelsäure, Säuresalze, Aminosäurehydrochloride, Natriumcitrate, Natriumcitratdihydrat, Dina-triumcitrat, Dinatriumdihydrogencitrat, Natriumsäurephosphat und Gemischen davon, vorzugsweise ist es Fumarsäure.

3. Brauseformulierung nach Anspruch 2, wobei die Menge an Fumarsäure im Bereich von 2-90 Gew.-% und vorzugsweise im Bereich von 2-60 Gew.-% der gesamten Formulierung liegt.

4. Brauseformulierung nach Anspruch 1, wobei das Alkalisierungsmittel ausgewählt ist aus der Gruppe bestehend aus Natriumglycincarbonat, Ammoniaklösung, Ammoniumcarbonat, Diethanolamin, Diisopropanolamin, Kaliumhydroxid, Kaliumbicarbonat, Kaliumcarbonat, Calciumcarbonat, Natriumbicarbonat, Natriumborat, Natriumcarbonat, Natriumhydroxid, Trolamin und Gemischen davon, vorzugsweise ist es Natriumglycincarbonat.

5. Brauseformulierung nach Anspruch 4, wobei die Menge an Natriumglycincarbonat im Bereich von 2-90 Gew.-% und vorzugsweise im Bereich von 2-60 Gew.-% der gesamten Formulierung liegt.

6. Brauseformulierung nach einem der vorstehenden Ansprüche in Form einer Tablette oder eines Dosierbeutels.

7. Brauseformulierung nach einem der vorstehenden Ansprüche, umfassend:
a) 0,01-10,0 Gew.-% Pramipexoldihydrochloridmonohydrat
b) 2,00-50,00 Gew.-% mikrokristalline Cellulose
c) 1,00-25,00 Gew.-% Polyvinylpyrrolidon
d) 0,01-2,00 Gew.-% Butylhydroxyanisol
e) 0,01-2,00 Gew.-% Butylhydroxytoluen
f) 2,00-60,00 Gew.-% Natriumglycincarbonat
g) 2,00-60,00 Gew.-% Fumarsäure
h) 0,10-2,00 Gew.-% Thaumatin
i) 0,50-10,00 Gew.-% Polyethylenglycol
j) 2,00-50,00 Gew.-% Natriumbicarbonat
k) 0,50-90,00 Gew.-% Wasser/Alkohol
l) 0,10-5,00 Gew.-% aromatisches Mittel

8. Brauseformulierung nach einem der vorstehenden Ansprüche, umfassend:
a) 0,01-10,0 Gew.-% Pramipexoldihydrochloridmonohydrat
b) 2,00-50,00 Gew.-% mikrokristalline Cellulose
c) 1,00-30,00 Gew.-% Crospovidon
d) 5,00-50,00 Gew.-% Erythritol
e) 1,00-30,00 Gew.-% Vinylpyrrolidin-Vinylacetatcopolymere
f) 2,00-60,00 Gew.-% Natriumglycincarbonat
g) 2,00-60,00 Gew.-% Fumarsäure
h) 0,10-2,00 Gew.-% Sucralose
i) 0,25-2,00 Gew.-% Polyethylenglycol
j) 0,10-5,00 Gew.-% aromatisches Mittel

9. Brauseformulierung nach einem der vorstehenden Ansprüche, umfassend:
a) 0,01-10,0 Gew.-% Pramipexoldihydrochloridmonohydrat
b) 5,00-50,00 Gew.-% Xylitol
c) 2,00-60,00 Gew.-% Natriumglycincarbonat
d) 2,00-60,00 Gew.-% Fumarsäure
e) 2,00-60,00 Gew.-% Natriumcitratdehydrat
f) 2,00-60,00 Gew.-% Weinsäure
g) 0,10-2,00 Gew.-% Sucralose
h) 0,10-2,00 Gew.-% Thaumatin
i) 0,10-5,00 Gew.-% aromatisches Mittel

10. Brauseformulierung nach einem der vorstehenden Ansprüche, umfassend:
a) 0,01-10,0 Gew.-% Pramipexoldihydrochloridmonohydrat
b) 5,00-50,00 Gew.-% Trehalose
c) 0,10-25,00 Gew.-% Polyvinylpyrrolidon
d) 2,00-60,00 Gew.-% Natriumglycincarbonat
e) 2,00-60,00 Gew.-% Fumarsäure
f) 2,00-60,00 Gew.-% Natriumcitrat
g) 0,10-2,00 Gew.-% Sucralose
h) 0,10-2,00 Gew.-% Mogrosid
i) 0,50-90,00 Gew.-% Wasser/Alkohol
j) 0,10-5,00 Gew.-% aromatisches Mittel

11. Brauseformulierung nach einem der vorstehenden Ansprüche, umfassend:
a) 0,01-10,0 Gew.-% Pramipexoldihydrochloridmonohydrat
b) 5,00-50,00 Gew.-% Trehalose
c) 0,10-25,00 Gew.-% Zitronensäureanhydrat
d) 2,00-60,00 Gew.-% Natriumglycincarbonat
e) 2,00-60,00 Gew.-% Fumarsäure
f) 2,00-60,00 Gew.-% Sorbitol
g) 0,10-2,00 Gew.-% Sucralose
h) 0,10-2,00 Gew.-% Natriumcyclamat
i) 0,10-5,00 Gew.-% aromatisches Mittel

## Revendications

1. Formulation effervescente comprenant du pramipexole ou un sel, solvate ou hydrate pharmaceutiquement acceptable de celui-ci et au moins un excipient pharmaceutiquement acceptable qui est choisi dans le groupe consistant en agents acidifiants, agents alcalinisants, charges, désintégrants, liants, lubrifiants, anti-oxydants, agents aromatiques et édulcorants ; et le rapport des agents acidifiants aux agents alcalinisants est entre 30:1 (p/p) et 1:30 (p/p), de préférence entre 15:1 (p/p) et 1:15 (p/p) et de façon davantage préférée est entre 3:1 (p/p) et 1:3 (p/p).

2. Formulation effervescente selon la revendication 1, dans laquelle l'agent acidifiant est choisi dans le groupe comprenant l'acide fumarique, l'acide tartrique, l'acide citrique, le monohydrate d'acide citrique, l'anhydrate d'acide citrique, l'acide adipique, l'acide ascorbique, l'acide acétique, l'acide chlorhydrique, l'acide malique, l'acide nitrique, l'acide phosphorique, l'acide nicotinique, l'acide acétyl salicylique, l'acide sulfurique, les sels d'acides, les chlorhydrates d'acides aminés, les citrates de sodium, le dihydrate de citrate de sodium, le citrate disodique, le dihydrogéno citrate disodique, le phosphate acide de sodium et leurs mélanges, étant de préférence l'acide fumarique.

3. Formulation effervescente selon la revendication 2, dans laquelle la quantité de l'acide fumarique se situe dans la plage de 2 à 90 % et de préférence de 2 à 60 % en poids de la formulation totale.

4. Formulation effervescente selon la revendication 1, dans laquelle l'agent alcalinisant est choisi dans le groupe comprenant le carbonate de glycine sodique, une solution ammoniacale, le carbonate d'ammonium, la diéthanolamine, la diisopropanolamine, l'hydroxyde de potassium, le bicarbonate de potassium, le carbonate de potassium, le carbonate de calcium, le bicarbonate de sodium, le borate de sodium, le carbonate de sodium, l'hydroxyde de sodium, la trolamine et leurs mélanges, étant de préférence le carbonate de glycine sodique.

5. Formulation effervescente selon la revendication 4, dans laquelle la quantité de carbonate de glycine sodique se situe dans la plage 2-90 % et de préférence de 2-60 % en poids de la formulation totale.

6. Formulation effervescente selon l'une quelconque des revendications précédentes, sous la forme d'un comprimé ou d'un sachet.

7. Formulation effervescente selon l'une quelconque des revendications précédentes, comprenant :
a) 0,01-10,0 % en poids de dichlorhydrate de pramipexole monohydraté ;
b) 2,00-50,00 % en poids de cellulose microcristalline ;
c) 1,00-25,00 % en poids de polyvinylpyrrolidone ;
d) 0,01-2,00 % en poids de butyl hydroxy anisole ;
e) 0,01-2,00 % en poids de butyl hydroxy toluène ;
f) 2,00- 60,00 % en poids de carbonate de glycine sodique ;
g) 2,00-60,00 % en poids d'acide fumarique ;
h) 0,10-2,00 % en poids de thaumatine ;
i) 0,50-10,00 % en poids de polyéthylène glycol ;
j) 2,00-50,00 % en poids de bicarbonate de sodium ;
k) 0,50-90,00 % en poids d'eau/alcool ;
l) 0,10-5,00 % en poids d'agent aromatique.

8. Formulation effervescente selon l'une quelconque des revendications précédentes comprenant :
a) 0,01-10,0 % en poids de dichlorhydrate de pramipexole monohydraté ;
b) 2,00-50,00 % en poids de cellulose microcristalline ;
c) 1,00-30,00 % en poids de crospovidone ;
d) 5,00-50,00 % en poids d'érythritol ;
e) 1,00-30,00 % en poids de copolymères vinylpyrrolidone-acétate de vinyle ;
f) 2,00-60,00 % en poids de carbonate de glycine sodique ;
g) 2,00-60,00 % en poids d'acide fumarique ;
h) 0,10-2,00 % en poids de sucralose ;
i) 0,25-2,00 % en poids de polyéthylène glycol ;
j) 0,10-5,00 % en poids d'agent aromatique.

9. Formulation effervescente selon l'une quelconque des revendications précédentes comprenant :
a) 0,01-10,0 % en poids de dichlorhydrate de pramipexole monohydraté ;
b) 5,00-50,00 % en poids de xylitol ;
c) 2,00-60,00 % en poids de carbonate de glycine sodique ;
d) 2,00-60,00 % en poids d'acide fumarique ;
e) 2,00-60,00 % en poids de dihydrate de citrate de sodium;
f) 2,00-60,00 % en poids d'acide tartrique ;
g) 0,10-2,00 % en poids de sucralose ;
h) 0,10-2,00 % en poids de thaumatine ;
i) 0,10-5,00 % en poids d'agent aromatique.

10. Formulation effervescente selon l'une quelconque des revendications précédentes comprenant :
a) 0,01-10,0 % en poids de dichlorhydrate de pramipexole monohydraté ;
b) 5,00-50,00 % en poids de tréhalose ;
c) 0,10-25,00 % en poids de polyvinylpyrrolidone ;
d) 2,00-60,00 % en poids de carbonate de glycine sodique ;
e) 2,00-60,00 % en poids d'acide fumarique ;
f) 2,00-60,00 % en poids de citrate de sodium ;
g) 0,10-2,00 % en poids de sucralose ;
h) 0,10-2,00 % en poids de mogroside ;
i) 0,50- 90,00 % en poids d'eau/alcool ;
j) 0,10-5,00 % en poids d'agent aromatique.

11. Formulation effervescente selon l'une quelconque des revendications précédentes comprenant :
a) 0,01-10,0% en poids de dichlorhydrate de pramipexole monohydraté ;
b) 5,00- 50,00 % en poids de tréhalose ;
c) 0,10-25,00 % en poids d'anhydrate d'acide citrique ;
d) 2,00-60,00 % en poids de carbonate de glycine sodique ;
e) 2,00-60,00 % en poids d'acide fumarique ;
f) 2,00-60,00 % en poids de sorbitol ;
g) 0,10-2,00 % en poids de sucralose ;
h) 0,10-2,00 % en poids de cyclamate de sodium ;
i) 0,10-5,00 % en poids d'agent aromatique.
